# EUROPEAN PATENT APPLICATION

(11) **EP 4 495 134 A1**
(43) Date of publication of application: **22.01.2025**
(21) Application number: 23721588.4
(22) Date of filing: 15.03.2023
(51) Int. Cl.: C07K 14/54

(54) **HUMAN INTERLEUKIN-2-DERIVED MUTEINS WITH SUPERAGONIST ACTIVITY**

(30) Priority: 18.03.2022 CU 20220020
(71) Applicant: Centro de Inmunologia Molecular, Playa, Habana 11300 (CU)
(72) Inventor: ROJAS DORANTES, Gertrudis, La Habana 10700 (CU); RELOVA HERNÁNDEZ, Ernesto, Batabanó Mayabeque 33400 (CU); CARMENATE PORTILLA, Tania, La Habana 12000 (CU); LEÓN MONZÓN, Kalet, La Habana 17100 (CU)
(74) Representative: V.O.
(86) International application number: PCT/CU2023/050001
(87) International publication number: WO 2023/174457

(57) **Abstract**

The present invention relates to the Biotechnology branch and is based on the identification of sets of IL-2 mutations in the vicinity of the interface with the receptor beta chain, through the selection of variants from filamentous phage libraries by affinity to the extracellular domain of the beta chain. Recombinant proteins derived from these variants show a very favourable developability profile, in terms of high expression levels, low tendency to aggregate, and high thermal stability. In addition, compared to the original unmutated IL-2 and to other described superagonist muteins, they have a higher capacity to stimulate immune effector populations carrying the dimeric IL-2 receptor and a higher anti-tumour activity *in vivo.*

## Description

### FIELD OF TECHNOLOGY

The present invention relates to the Biotechnology branch, particularly to a family of mutated proteins (muteins) derived from human Interleukin-2 (IL-2), which have an increased binding capacity to the beta chain of the IL-2 receptor and a favourable developability profile, conferring potent superagonist activity in vitro and in vivo.

### BACKGROUND

While IL-2 was originally described as a T-cell growth factor (Smith, K.A. Immunol. Rev. 51: 337-357, 1980), it is now known as a regulator with dual functions in the immune response (Malek, T.R. Annu. Rev. Immunol. 26: 453-479, 2008; Hoyer K.K. et al., Immunol. Rev. 226: 19-28, 2008), which promotes or negatively modulates effector functions of the immune system. Although the constitutive presence of the dimeric intermediate affinity beta/gamma receptor on effector cells of the immune system mediates the potent immunostimulatory functions of IL-2, the existence of high levels of the alpha chain on the surface of regulatory T cells endows them with a high-affinity trimeric receptor, which allows preferential use of the cytokine by this cell population over other immune cells (Malek, T.R. and Castro, I. Immunity. 33: 153-165, 2010). As a consequence, in the physiological state the essential role of IL-2 is the maintenance of immune tolerance (Malek, T.R. and Bayer, A.L. Nat. Rev. Immunol. 4: 665-674, 2004) through the stimulation of regulatory T cells.

The functional dichotomy of IL-2 has opened wide possibilities for its exploitation to produce opposing therapeutic effects on the immune system and to modulate the immune response in either direction in different scenarios. Its use in high doses has demonstrated its capacity of immunopotentiation, useful for stimulating anti-tumour responses (Klapper, J.A. et al., Cancer. 113: 293-301, 2008). Beyond pharmacological manipulation, protein engineering modification of the molecule itself has led to selective modulation of interactions with the different receptor subunits, and consequent alteration of the natural balance between immunostimulatory and immunoregulatory functions. For example, mutated variants of IL-2 with superagonist properties have been described, in which increased binding capacity to the beta subunit leads to potent stimulation of effector CD8+ and NK T cells and a strong anti-tumour effect (Levin, A.M. et al., Nature. 484: 529-533, 2012). As the interaction with the dimeric beta/gamma receptor present at high levels on effector cells is strengthened, the relative contribution of the alpha subunit characteristic of regulatory T cells decreases and therefore molecules with functions biased towards immunopotentiation of the immune response are created. These muteins, among which the most studied is the so-called H9 superkine, are derived from the selection from libraries displayed on the surface of yeast cells (Levin, A.M. et al., Nature. 484: 529-533, 2012). Such a technology platform has dominated IL-2 *in vitro* evolution procedures (Rao, B.M. et al., Protein Eng. 16:1081-1087, 2003). Although IL-2 was displayed on filamentous phages more than two decades ago (Buchli, P.J. et al., Arch. Biochem. Biophys. 339: 79-84, 1997; Vispo, N.S. et al, Immunotechnology. 3: 185-193, 1997), this technology has so far not been useful in finding new sets of mutations that modulate the interactions of IL-2 with its receptors.

Modifying the functional properties of cytokines by introducing individual mutations or discrete sets of mutations often results in deleterious effects on the stability and solubility of the molecules, which negatively impact their expression levels as recombinant proteins, their developability profile as therapeutic agents, and ultimately their biological activity. These limitations have led to more drastic approaches to *de novo* redesign of stable agonists based on extensive *in silico* optimisation and receptor affinity selection (Silva, D-A. et al., Nature. 565: 186-191, 2019). These molecules deviate greatly from the primary sequence of natural cytokines, so their potential immunogenicity and unwanted interactions within the organism are of concern.

Surprisingly, the inventors of the present invention defined, from the directed evolution of human Interleukin-2 displayed on filamentous phages, discrete sets of mutations with regularities not previously described or predictable from structural analysis of human IL-2, the introduction of which increases the ability of the cytokine to bind to the beta subunit of its receptor and thus to stimulate immune system cells carrying the dimeric IL-2 receptor. The IL-2 variants thus modified are distinguished from other superagonists described in the prior art by improved developability in terms of expression levels, low tendency to aggregate, and thermal stability, which confer greater superagonist capacity and anti-tumour activity *in vivo.* This finding lays the foundation for the exploitation of these variants on an industrial scale to obtain therapeutic products, useful in the immunotherapy of cancer, immunodeficiencies and in the *ex vivo* stimulation of immune cells for adoptive immunotherapy protocols.

### BRIEF DESCRIPTION OF THE INVENTION

In one embodiment the present invention is based on the identification of sets of mutations located in the vicinity of the IL-2 receptor beta chain binding interface from libraries of human IL-2 variants displayed on filamentous phages. Phage selection for affinity to the recombinant extracellular domain of the beta chain leads to the isolation of variants with increased binding capacity. The sets of mutations identified by this route are diverse, but have regularities that determine the properties of the selected variants. In particular, they are distinguished by the presence of changes in at least two of the positions 81, 83, 84 and 87 of the primary amino acid sequence of IL-2, resulting in a net negative charge of the 81-87 segment equal to or greater than -2. Another distinctive feature is the presence preferentially of the I92L substitution or the original Ile residue at position 92, with no other substituents at that position. The regularities of the sequences are shown in Table 1 (SEQ ID NO. 1-13).

Optionally, position 80 can be mutated to amino acids Phe, Val, Met or Ile, position 82 to Ala, position 85 to Ile, Val or Met, position 86 to Val, position 89 to Leu or Met and position 93 to Ile or Leu (SEQ ID NO. 14-26).

In another embodiment the present invention comprises the genetic construction of new recombinant molecules through the introduction of the previously identified mutations, alone or combined with K35E, K35D and K35Q mutations (SEQ ID NO. 27-52) and/or other substitutions known to affect the properties of IL-2 (SEQ ID NO. 127-178), and the fusion to genes coding for proteins of interest such as antibodies, their domains and fragments, albumin, filamentous phage capsid proteins, cytokines and others.

The subject of the present invention are also the recombinant proteins produced from the genetic constructions described above in expression systems in prokaryotic host cells such as *E.coli* and eukaryotic host cells such as HEK293, CHO, NS0, insect and yeast cells. These proteins possess an increased binding capacity to the beta-chain of the receptor, a feature for which they were selected and which leads to potent superagonist activity on cells carrying the dimeric beta/gamma IL-2 receptor. Unlike superkine H9 and other similar molecules described in the prior art, these muteins are further distinguished by a favourable developability profile (high expression levels, low tendency to aggregate and high thermal stability). The overall properties of the muteins obtained as part of the present invention give them better developability and a greater capacity for stimulation of immune system effector cells and anti-tumour activity *in vivo*, compared to the original IL-2 and to superkine H9. The subject of the present invention are also the pharmaceutical compositions comprising the muteins described herein in a concentration range of 1 mg/ml to 20 mg/ml and a pharmaceutically suitable excipient.

In another embodiment, is the subject of the present invention, the use of muteins and recombinant molecules derived from them as drugs for cancer immunotherapy, immunodeficiencies, and in *ex vivo* stimulation of immune cells for adoptive immunotherapy protocols.

### DETAILED DESCRIPTION OF THE INVENTION

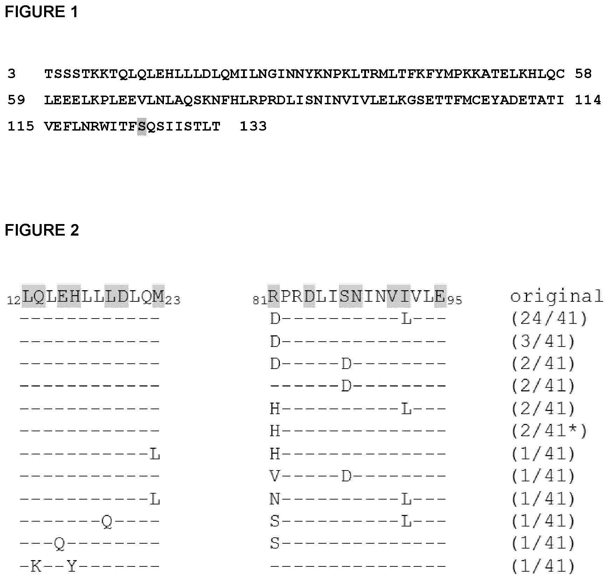

The present invention is based on the identification of sets of mutations in the IL-2 sequence shown in Figure 1, to which the C125S change was introduced in contrast to the wild-type IL-2 sequence (shaded in grey). These sets of mutations are located in the vicinity of the binding interface of human IL-2 to the beta chain of its receptor, and were selected from filamentous phage libraries. These sets of mutations increase the ability of the cytokine to bind to the beta subunit of the receptor, and thus stimulate immune system cells carrying the dimeric IL-2 receptor. Diverse recombinant proteins derived from the mutated IL-2 variants are constructed, including additional substitutions and fusion to other protein domains. The developability profile of the modified molecules is shown to be favourable, due to their high expression levels, low tendency to aggregate and high thermal stability, which confer enhanced superagonist capacity and anti-tumour activity *in vivo.* The superiority of the properties of the new muteins over the equivalent recombinant molecules, obtained in the same format and expression system from nonmutated IL-2 and H9 superkine already known as part of the prior art, is evidenced.

### Selection of mutation sets that enhance interaction with the beta chain of the receptor from libraries of IL-2 variants displayed on filamentous phages.

Selection of human IL-2 derived polypeptides with mutations leading to increased binding capacity to the beta-subunit of the receptor can be made from libraries of IL-2 variants displayed on filamentous phages. Genes corresponding to these variants are inserted into phagemid-type expression vectors (fused to one of the genes encoding for the capsid proteins of filamentous phages) and used for the production of viral particles that expose the protein variants on their surface. Libraries can include varying degrees of diversification (soft or hard randomisation) along the entire sequence or at a pre-defined set of positions. Each of the original residues at these positions can be replaced by a mixture of the 20 amino acids or by a chosen subset of residues. Diversification can be achieved through random or site-directed mutagenesis.

Selection of phages with the highest beta-chain binding capacity is based on incubation of phage mixtures from the libraries on a support on which a recombinant protein containing the extracellular domain of the IL-2 receptor beta subunit is immobilised, removal of unbound phages by washing and elution of bound phages. Several similar screening cycles can be performed. Analysis of the DNA sequences inserted into the selected phages reveals regularities that allow identification of the most abundant substitutions (and combinations thereof) potentially associated with increased binding capacity. Screening of an initial library can be used to identify hotspots that modulate the interaction, followed by further exploration of these hotspots and their vicinity through secondary libraries.

The ability of variants to interact with the beta-subunit can be assessed in binding assays such as solid-phase enzyme-linked immunosorbent assays (ELISA) or similar phage assays. To segregate direct effects on beta-chain binding capacity (which are the intended effects) from possible changes in the display levels on phage of the IL-2 variants, the signals obtained in the binding assay can be normalised to the signals detected with a molecule that reacts equivalently with all variants, such as an antibody directed against a common tag peptide fused to them.

As a result of the selective processes, different variants with different sequences are obtained, but which comply with certain regularities. The first is the presence of nonsilent mutations (with change of the encoded amino acid) in at least two of the selected positions within the group comprising residues 81, 83, 84 and 87 of the primary sequence of IL-2, resulting in a net negative charge of the 81-87 segment equal to or greater than -2. Another regularity is the frequent presence of the I92L mutation (predominant), with no other substituents at that position. The various combinations of residues that fulfill these regularities are illustrated in Table 1, which comprises thirteen classes of muteins (SEQ ID NO. 1-13). These changes can optionally be accompanied by other modifications at positions 80, 82, 85, 86, 89 and 93 (SEQ ID NO. 14-26). Such combinations of mutations lead to increased reactivity of the IL-2 variants on phage with the beta-subunit of the receptor, compared to the original IL-2.

**Table 1. Primary sequence regularities of the thirteen classes of mutated human IL-2 variants identified as part of the present invention.**

| **IL-2 variants** | **pos. 81** | **pos. 83** | **pos. 84** | **pos. 87** | **pos. 92** | **Net charges (segment 81-87)** |
|---|---|---|---|---|---|---|
| **Original (non mutated)** | Arg | Arg | Asp | To be | Ile | +1 |
| **Class A Muteins** (SEQ ID NO. 1) | Group I aa (basic) | Group II aa (acidic) | Group II aa (acidic) | Group II aa (acidic) | Leu/Ile | -2 |
| **Class B** (SEQ ID NO. 2) | Group II aa (acidic) | group I aa (basic) | group II aa (acidic) | group II aa (acidic) | Leu/Ile | -2 |
| **Class C** (SEQ ID NO. 3) | group III aa (neutral) | group III aa (neutral) | group II aa (acidic) | group II aa (acidic) | Leu/Ile | -2 |
| **Class D** (SEQ ID NO. 4) | group II aa (acidic) | group III aa (neutral) | group II aa (acidic) | group III aa (neutral) | Leu/Ile | -2 |
| **Class E** (SEQ ID NO. 5) | group III aa (neutral) | group II aa (acidic) | group II aa (acidic) | group III aa (neutral) | Leu/Ile | -2 |
| **Class F** (SEQ ID NO. 6) | group II aa (acidic) | group II aa (acidic) | group III aa (neutral) | group III aa (neutral) | Leu/Ile | -2 |
| **Class G** (SEQ ID NO. 7) | group II aa (acidic) | group III aa (neutral) | group III aa (neutral) | group II aa (acidic) | Leu/Ile | -2 |
| **Class H** (SEQ ID NO. 8) | group III aa (neutral) | group II aa (acidic) | group III aa (neutral) | group II aa (acidic) | Leu/Ile | -2 |
| **Class I** (SEQ ID NO. 9) | group II aa (acidic) | group III aa (neutral) | group II aa (acidic) | group II aa (acidic) | Leu/Ile | -3 |
| **Class J** (SEQ ID NO. 10) | group III aa (neutral) | group II aa (acidic) | group II aa (acidic) | group II aa (acidic) | Leu/Ile | -3 |
| **Class K** (SEQ ID NO. 11) | group II aa (acidic) | group II aa (acidic) | group II aa (acidic) | group III aa (neutral) | Leu/Ile | -3 |
| **Class L** (SEQ ID NO. 12) | group II aa (acidic) | group II aa (acidic) | group III aa (neutral) | group II aa (acidic) | Leu/Ile | -3 |
| **Class M** (SEQ ID NO. 13) | group II aa (acidic) | group II aa (acidic) | group II aa (acidic) | group II aa (acidic) | Leu/Ile | -4 |

The thirteen classes of mutated variants of IL-2 that meet the regularities identified as part of the present invention are named with the letters of the Latin alphabet, from A to M).

Group I (basic) amino acids (aa) comprise Arg, Lys and His residues.

Group II (acidic) aa's comprise the Asp and Glu residues.

Group III (neutral) aa's include the residues Ala, Asn, Gly, Gln, Ile, Leu, Met, Phe, Pro, Ser, Thr, Val, Trp and Tyr. Cys is excluded from the possible substituents.

### Introduction of identified mutations in recombinant IL-2-derived proteins and characterisation of their binding properties

Once sets of mutations leading to increased interaction with the beta-subunit of the receptor have been identified, they are introduced by site-directed mutagenesis into IL-2 coding genes contained in different expression vectors designed for modification of bacterial host cells, yeast cells, insect-derived or mammalian-derived cell lines (both plasmid and viral vectors). These sets of mutations may match the combinations of substitutions isolated directly from the phage array, but novel combinations can also be designed between them.

A substitution at position 35 of the IL-2 primary sequence can additionally be introduced by amino acids E, D or Q (SEQ ID NO. 27-52), whose beneficial effect on secretion levels, aggregation resistance and biological activity of IL-2-derived molecules is known (WO2018/0910003; Rojas, G et al., Sci. Reports. 9: 800, 2019). From these sequences of IL-2 muteins, other recombinant molecules can be constructed through genetic fusion with proteins such as albumin, Fc regions and variable domains of antibodies, whole antibodies and other cytokines.

The molecules described above can be obtained from:
a. transformation of host bacteria (such as *Escherichia coli*) or yeast cells with the corresponding plasmids.
b. transfection of insect cells with baculovirus-derived vectors or plasmids.
c. transient or stable transfection of host cells such as HEK-293, CHO, NS0 and other mammalian-derived cells with the corresponding plasmids, as well as transduction of these host cells with viral vectors containing the genes of interest.

The increased binding capacity of the muteins to the beta-subunit of the receptor can be demonstrated by immunochemical methods such as ELISA or surface plasmon resonance measurement (BIAcore^{™}). The binding capacity of the recombinant proteins derived from the mutated variants described in the present invention to the beta chain of the IL-2 receptor is higher than that of their counterparts based on the original IL-2.

### Characterisation of the developability profile of muteins and recombinant proteins derived from them.

The set of properties grouped under the term "developability profile" includes biophysical characteristics such as aggregation tendency and thermal stability, as well as secretion levels of the proteins of interest in different recombinant protein production systems. All of these have a direct influence on the feasibility of developing production processes and obtaining biological products suitable for their final application.

Aggregation tendency is studied by molecular exclusion chromatography which detects the presence and proportion of monomeric species and aggregates of different sizes. The existence of aggregates resistant to denaturation is demonstrated by electrophoresis in SDS-polyacrylamide gels.

Thermal stability is characterised by comparing molecules treated at high temperatures with their untreated counterparts to detect the change in their properties associated with thermal stress.

Secretion levels can be measured in different expression systems based on genetically modified host cells, using methods that allow quantification of secreted molecules in supernatant samples. Quantification of purified proteins from a given volume of supernatant allows direct determination of yields.

The mutated variants of human IL-2 described as part of the present invention and the recombinant molecules derived from them are distinguished by a favourable developability profile, characterised by higher levels of secretion as soluble proteins from host cells, a reduced tendency to aggregate and increased thermal stability, compared to their counterparts containing the original IL-2 or H9 superkine sequence. The presence of the I92L change is associated with enhancement of these properties. In contrast, all variants containing the I92F change, present in H9 superkine and other IL-2-derived muteins with increased receptor beta-chain binding capacity (Levin, A.M. et al., Nature. 484: 529-533, 2012; WO2020/260270 A1), show unfavourable developability properties, such as decreased levels of secretion by host cells, high propensity for aggregation, and poor thermal stability.

The exclusion of the I92F change from the muteins claimed as part of the present invention, and the preferential introduction of a Leu residue at that position are key to obtaining the favourable developability profile that distinguishes this group of muteins and confers an advantage over molecules with similar receptor beta chain binding properties described as part of the prior art.

### Demonstration of superagonist character of muteins on cells carrying the dimeric beta/gamma IL-2 receptor in vitro and in vivo

The superagonist activity of the new molecules is evidenced by *in vitro* stimulation of purified cells expressing the dimeric beta/gamma IL-2 receptor, the proliferation of which is detected by flow cytometry methods.

To demonstrate the *in vivo* effect of muteins on the expansion of immunological effector populations, experimental animals are treated with the muteins and the populations of interest are also monitored by flow cytometry.

The recombinant proteins derived from the muteins described as part of the present invention show an increased ability to stimulate *in vitro* cells carrying the dimeric IL-2 receptor relative to those containing the original IL-2 and to an extent not inferior to that of the H9 superkine. Their ability to stimulate cell populations of this type *in vivo* exceeds that of both the proteins based on the original IL-2 and the reported H9 variant.

### Demonstration of anti-tumour effect of muteins

The anti-tumour effect of recombinant proteins derived from muteins can be demonstrated in animal models of malignant tumours. Administration of these proteins to animals inoculated with syngeneic transplantable tumour cell lines can prevent tumour formation, slow tumour growth or reduce metastasis formation in various organs.

The recombinant proteins derived from the muteins described as part of the present invention have anti-tumour effects as described in experimental tumour models, in a magnitude superior to their counterparts containing the original IL-2 and even exceed in some experimental systems those including superkine H9 reported in the prior art.

### Pharmaceutical compositions

The muteins subject of the present invention can be found as an active ingredient, forming part of different pharmaceutical compositions suitable for themselves and a pharmaceutically acceptable vehicle. The concentrations of the active ingredient in such pharmaceutical compositions are in the range of 1 mg/ml to 20 mg/ml.

Pharmaceutically suitable vehicles include, but are not limited to: saline solution, pH neutral phosphate buffered saline solution and the like. Other buffering agents, dispersing agents and non-toxic inert substances suitable for delivery to a patient may be included in the compositions of the present invention. The compositions may be solutions suitable for administration and are normally sterile and free of undesirable particles.

### Therapeutic application and treatment methods

This invention, further, includes possible therapeutic applications of the muteins described herein with the aim of enhancing the natural or vaccine-induced immune response in diseases such as cancer or immunodeficiencies where effector T cells are particularly relevant. In addition, the use in the *in vitro* expansion of T and NK cells for adoptive transfer therapies is envisaged.

For therapeutic use, the muteins of the present invention should be administered to a subject carrying the disease independently or in combination with other muteins or with other substances that facilitate or potentiate their therapeutic action. The route of administration may be any of the routes of administration described by the prior art for parenteral administration of drugs. It may preferably be administered intravenously, intramuscularly, subcutaneously or intratumorally.

The muteins of the present invention can be used in combination with therapeutic vaccines for cancer.

To obtain the desired therapeutic effect, the muteins of the present invention should be administered at doses high enough to ensure a concentration of the muteins in the lymph node or peripheral site relevant to the disease under study, which is in the range of concentrations for which the muteins show an immune-stimulating effect. The dose in question should therefore be adjusted for the type of disease and route of administration under study. For example in the case of tumour therapy, the dose should be adjusted to achieve concentrations of the mutein within the tumour and/or in the loco-regional lymph node that ensure stimulation of an anti-tumour immune response. Dose ranges to be explored can include from 20 µg/kg body weight to 200 µg/kg body weight. For those applications where mutein replaces a traditional native IL-2 therapy, the dose of mutein to be used should be lower or equivalent in activity (determined using CTLL-2 line assay) to that traditionally used for native IL-2.

The number of administrations to be applied should also be adjusted to the biodistribution of the mutein in question. In general, the effective concentrations referred above should be sustained for anywhere from 2 days to 30 consecutive days. Note for example that if mutein is fused to a carrier protein, the frequency of administrations may decrease and the dose of mutein should be adjusted accordingly, in this case the dose range varies from 100 µg/kg bw to 1mg/kg bw. For applications where native IL-2 is substituted, the administration schedule of mutein may be similar to that applied in traditional therapy.

Therapeutic action should be understood as the total or partial remission of the symptoms of the disease. In cancer, a decrease in tumour volume or an increase in relapse time will be, among others, the criteria for remission of the disease.

The polypeptides of the invention are particularly useful in the therapy of tumours such as among others melanomas, kidney tumours, ovarian, breast and lung tumours. They may also be useful in immunodeficiencies such as HIV and severe combined immunodeficiency.

### BRIEF DESCRIPTION OF THE FIGURES

**Figure 1****.** Primary amino acid sequence of the original IL-2 (T3-T133) taken as the basis for the construction of the muteins on filamentous phages.
**Figure 2****.** Sequences of the modified segments (12-23 and 81-87) in the soft randomisation library of the IL-2 interface with the beta subunit of the receptor.
**Figure 3****.** Representation of the global profile of sequences isolated from the S1 library after phage targeting of the recombinant extracellular domain of the beta subunit of the receptor.
**Figure 4****.** Charges of segment 81-87 in selected variants of the S1 library.
**Figure 5****.** Increased binding capacity of the phage mixture selected from the S1 library.
**Figure 6****.** Sequences of the modified segment (80-93) of IL-2 in the clones selected from the S2 library.
**Figure 7****.** ELISA-determined reactivity of IL-2 variants on filamentous phages selected from the S2 library.
**Figure 8****.** Primary amino acid sequence of IL-2 (A1-T133).
**Figure 9****.** Yield of proteins fused to human Fc in the transient transfection system of HEK-293T cells adapted to grow in suspension.
**Figure 10****.** Productivity of stable clones producing fusion proteins of IL-2 muteins to human IgG1 Fc.
**Figure 11****.** Binding capacity of human Fc fusion proteins derived from IL-2 muteins selected from the S1 library to the beta chain of the receptor.
**Figure 12****.** Binding capacity of human Fc fusion proteins derived from IL-2 muteins selected from the S2 library to the beta chain of the receptor.
**Figure 13****.** Relative reactivity against the beta chain after treatment of fusion proteins at 50°C.
**Figure 14****.** *In vivo* expansion of the activated effector T cell population (CD8+CD44hi CD122hi). The number of cells in each mouse is shown.
**Figure 15****.** *In vivo* expansion of the regulatory T cell population (CD4+CD25+Foxp3+). The number of cells in each mouse is shown.
**Figure 16****.** Anti-metastatic effect of human LALA Fc fusion proteins containing IL-2 muteins.
**Figure 17****.** Anti-metastatic effect of fusion proteins containing combined muteins with increased binding capacity to the beta chain of the receptor and decreased binding capacity to the alpha chain in the MB16/F0 model.

### EXAMPLES

### Example 1. Construction of a soft randomisation library of phage-displayed human IL-2 interface with the IL-2 receptor beta subunit and selection of mutated variants therefrom.

For the initial identification of positions that modulate the interaction of IL-2 with the beta subunit of the receptor we used, in contrast to the random diversification employed in previous work (Levin, A.M. et al., Nature. 484: 529-533, 2012), a soft randomisation strategy focused on the interface of interest. The genetic construct described (Rojas, G. et al., Immunobiology 218: 105-113, 2013; Rojas, G. and Carmenate, T., Methods Mol. Biol.1701: 535-560, 2018) containing the gene coding for human IL-2 (residues T3-T133, C125S, Figure 1) fused to the filamentous bacteriophage M13 protein III gene, was used. A 7x10⁷ member library was constructed by Kunkel mutagenesis (Kunkel, T., Proc. Natl. Acad. Sci. USA 82: 488-492, 1985) with two degenerate antisense mutagenic oligonucleotides. The oligonucleotides introduced limited variability at 14 positions (seven each in amino acid segments 12-23 and 81-95 of IL-2) (Figure 2). The residues to be mutated were chosen because of their location at the interface with the beta chain (< 5Å) in the structure of the IL-2/receptor complex (PDB code 2B5I). These oligonucleotides included 90% of the nucleotide complementary to the original at each position of the triplets to be diversified, and the remaining 10% of a mixture of the other three nucleotides.

Library phages were produced according to a protocol described (Marks, J.D. et al., J. Mol. Biol. 222: 581-597, 1991), and phages with the ability to bind to the surface of immunotubes coated with the recombinant extracellular domain of the human beta subunit were selected. Sequencing of a sample of the phage obtained after three cycles of selection revealed the presence of mutated variants as shown in Figure 2. The first line of the figure shows the original IL-2 (with the residues to be modified shaded in grey), and below the variants isolated after three cycles of selection. Dashes indicate the conservation of the original IL-2 residue at a given position. The frequency of each variant in the sample of 41 clones analysed after selection is shown in brackets. The asterisk (*) represents the existence of other mutations outside the intentionally modified region.

The amino acids that exhibited the highest frequency of mutations in this panel were R81 (preferentially substituted by D), I92 (I92L) and S87 (S87D). These three positions were chosen as hotspots that modulate the interaction with the beta subunit of the receptor.

### Example 2. Combinatorial exploration of the vicinity of the identified of hotspots through secondary phage libraries

Two secondary libraries (S1 and S2) were constructed by techniques similar to those described in the previous example. Library S1 included the total randomisation of the hot spots R81 and S87 and the neighbouring (also solvent-exposed) residues R83 and D84, as well as the replacement of the adjacent residue P82 by the Pro/Ala mixture. The S2 library included, in addition to the above modifications, the replacement of residues I92 (hot spot) and other hydrophobic core amino acids close to the interface with the beta chain (L80, L85, I86, I89 and V93), by a mixture of hydrophobic residues (Phe/Ile/Met/Leu/Val).

While preceding work had addressed the diversification of the hydrophobic core of IL-2 (Levin, A.M. et al., Nature. 484: 529-533, 2012), the randomisation of protuberant and highly exposed to the solvent residues to positively modulate IL-2 interaction with the beta chain is novel.

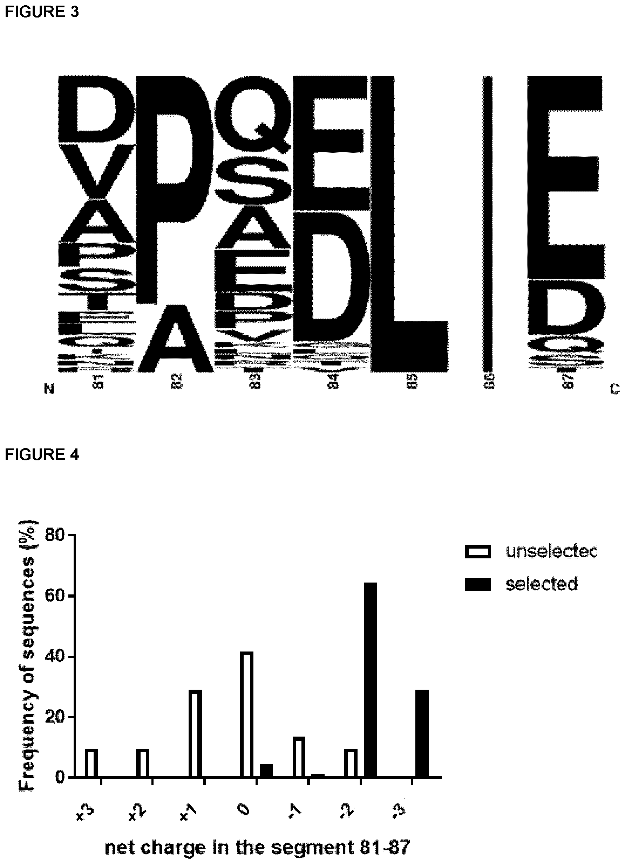

Sequencing of a sample of clones obtained from the S1 library, after three cycles of selection on immunotubes coated with the recombinant extracellular domain of the beta subunit of the receptor, revealed a wide diversity. Forty-eight unique sequences (SEQ ID NO. 53-100) were found, none repeated more than three times, but fulfilling certain regularities as shown in Figure 3, where the letters represent the amino acids encoded at each position of the 81-87 segment. The size of each letter is proportional to the frequency of occurrence of the corresponding residue at a given position.

The basic Arg residues at positions 81 and 83 were replaced by several amino acids, including Asp and Glu (acidic). The acidic character of residue D84 was retained for the most part or preferentially replaced by Glu. The amino acid S87 was frequently replaced by both Glu and Asp. This selection pattern revealed the possible importance of the accumulation of negative charges on the 81-87 segment of IL-2 for the increased interaction with the beta chain. While IL-2 has a net positive charge (+1) in this area and the charge of the H9 variant described in the prior art is -1, variants with net negative charges of -2 and -3 in segment 81-87 predominated among the new variants selected from the S1 library, even though that segment in the unselected library was largely neutral due to randomisation (Figure 4).

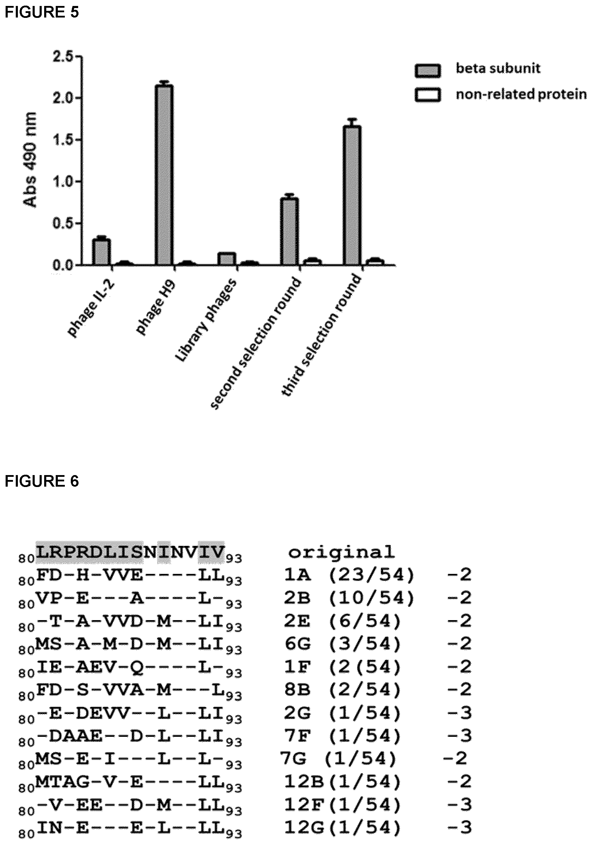

The reactivity of phage mixtures obtained after two and three cycles of selection was assessed by ELISA on plates coated with the recombinant extracellular domain of the beta subunit of the receptor and with the unrelated protein bovine serum albumin. Bound phages were detected with an anti-phage antibody conjugated to horseradish peroxidase. Phages bearing the original unmutated IL-2 and its H9 variant (superkine) were used as controls. The functional result of the selection was an increased reactivity with the beta-chain of the phage mixture (Figure 5).

Figure 6 shows the sequences obtained after three cycles of selection of the phage from the S2 library on the recombinant extracellular domain of the beta subunit of the receptor(SEQ ID NO. 100-112). The first line shows the original starting IL-2 (with the diversified residues shaded in grey), and below the variants isolated after three cycles of selection on the recombinant extracellular domain of the immobilised receptor beta subunit. Dashes indicate the conservation of the original IL-2 residue at a given position. The number of times each variant was repeated within the sample of 54 clones analysed is shown in brackets. The numbers at the end of each line represent the net charge of the 81-87 segment in each selected variant.

As shown in the Figure 6, twelve different sequences were obtained (SEQ ID NO. 100-112), with different relative abundance. The selected variants were distinguished by the preferential presence of the I92L substitution; the disappearance of the original Arg residues at positions 81 and 83; the frequent finding of acid residues at positions 81, 83 and 87; and the preservation of the acidic character of residue D84 (only substituted by Glu). This pattern of sequences resulted in a considerable net negative charge (-2 or higher) in segment 8187. The above data reinforced the importance of electrostatic interactions in optimising binding to the beta-subunit. While there were overlaps between the sequences selected from the S2 library and the H9 superkine described in the prior art (the eventual appearance of the L80F, R81D, L85V and I86V changes), there were marked differences between them. The sequences isolated from the S2 library mostly included the I92L change, as opposed to the I92F mutation present in H9. The latter retains R83, while in the new variants this residue is substituted. S87 is also conserved in H9, but is frequently replaced by acidic amino acids in the new variants. The net charge of segment 81-87 is more negative in the library variants (-2, -3) than in H9 (-1). The results demonstrated the possibility of obtaining human IL-2 variants with an increased beta chain binding capacity through different structural solutions and different from that of H9.

The binding capacity of the new variants to the extracellular domain of the IL-2 receptor beta subunit was assessed by ELISA. For this purpose, plates were coated with the recombinant extracellular domain of the IL-2 receptor beta subunit. The bound phages were detected with an anti-M13 antibody conjugated to horseradish peroxidase. Simultaneously, their binding to a plate coated with the monoclonal antibody 9E10 directed against the *c-myc* peptide that is fused to the proteins presented on the phages was determined to establish the display levels of each. The ratio between the signals obtained with both coating molecules (relative reactivity) is plotted as a measure of the intrinsic binding capacity of each variant to the beta-subunit. The original IL-2 and H9 superkine, also displayed on phage, were used as controls.

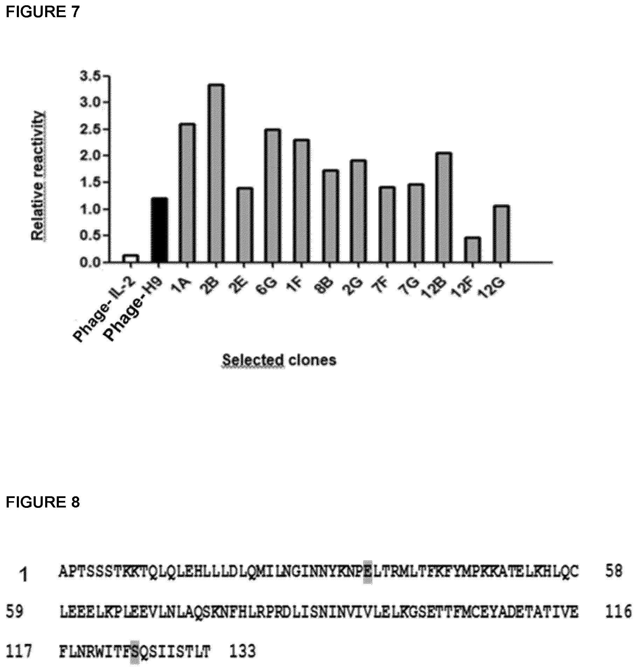

Figure 7 shows an increased binding capacity of these variants in contrast to human IL-2 and in some cases also to H9 displayed on phage.

### Example 3. Design of fusion proteins derived from novel IL-2 variants and production by transient transfection

Characterisation of the phage-selected variants as soluble proteins was performed in the format of fusion proteins to the N-terminal end of an Fc region of human IgG1 (SEQ ID NO. 113). The expression system of choice was transient transfection of HEK-293T cells adapted to grow in suspension (Jäger et al., BMC Biotechnol. 13: 52, 2013). For production in eukaryotic hosts, the first two amino acids (Ala-Pro) of IL-2, absent in the molecules diplayed on phage, were added and the K35E mutation, with a demonstrated beneficial effect on IL-2 developability (Rojas, G. et al., Sci. Reports. 9: 800, 2019), was introduced. Figure 8 shows the primary sequence of IL-2 taken as the basis for production in eukaryotic cells, to which the relevant changes were introduced at the interface with the beta chain. These changes were designed based on the screening results of the initial soft-randomised library of the interface and the secondary libraries S1 and S2.

Two fusion proteins with frequently selected mutations (R81D/R81V, R83Q/R83E and S87E) and a net negative charge of -3 in segment 81-87 were designed from the S1 library products. These variants are hereafter referred to as ₈₁DPQDDLIE₈₇ (SEQ ID NO. 114) and ₈₁VPEDLIE₈₇ (SEQ ID NO. 115) for their primary sequence in this segment. The introduction of the additional I92L change (repeatedly selected from the initial screening of the soft randomisation library) resulted in two further fusion proteins: ₈₁DPQDLIE₈₇ +I92L (SEQ ID NO. 116) and ₈₁VPEDLIE₈₇ +I92L (SEQ ID NO. 117) .

Among the molecules selected from the S2 library, the variants displayed on phage 1A and 2B were chosen due to their high reactivity against the beta chain, their recurrent presence among the selected clones and the net negative charge of -2 in the 81-87 segment. Two fusion proteins were constructed from them, hereafter referred to as ₈₀FDPHDWE₈₇+I92L+V93L (SEQ ID NO. 118) and ₈₀VPPEDLIA₈₇+I92L (SEQ ID NO. 119) respectively because of their sequences at the interface with the beta chain.

The variant ₈₀VPPEDLIA₈₇+I92L (SEQ ID NO. 119) does not share any mutation with the H9 superkine, thus representing a new structural solution for the increased interaction with the beta subunit. With the intention of optimising it, another variant was designed (₈₀VPPEDLIE₈₇+I92L) (SEQ ID NO. 120) , which also incorporates the S87E mutation, which was frequently selected from the library and increases the net negative charge of the interface (-3).

In contrast, the sequence of other clones selected from the S2 library does overlap with that of the H9 superkine, so combining the changes present in H9 with the novel distinguishing features of the variants selected from our libraries was explored as a possible avenue for optimisation. The L80F, R81D, L85V and I86V changes in H9 (also selected from the phage library) were retained, but R83 was replaced by Ala or Glu (both frequently selected from the phage library) and the S87E change (recurring mutation among the new variants) was introduced. As position 92 on H9 is occupied by a Phe residue and the I92L change was mostly found in the selected variants, both residues (Phe and Leu) were evaluated at this position. The variants designed and constructed were:
₈₀FDPADWE₈₇+I92L (SEQ ID NO. 121)
₈₀FDPADWE₈₇+I92F (SEQ ID NO. 125)
₈₀FDPEDWE₈₇+I92L (SEQ ID NO. 122)
₈₀FDPEDWE₈₇+I92F (SEQ ID NO. 126)

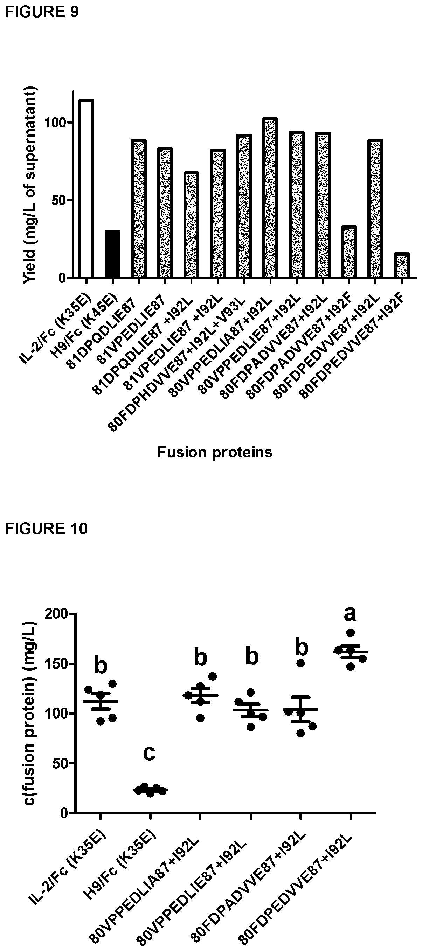

Figure 9 illustrates the mass yields of protein A affinity purification from 1L of culture supernatant of the different fusion proteins in a transient transfection experiment of HEK-293T cells in suspension. The decreased secretion levels of the H9 superkine-containing fusion proteins (30 mg/L supernatant), as well as the other two engineered muteins that include the I92F change in their sequence (<33 mg/L) were striking. In contrast, the rest of the muteins designed from the phage library screening results were produced at higher levels (>67 mg/L). This result was a first evidence of the favourable developability profile associated with the new muteins, and suggested the deleterious effect of the I92F mutation present in the H9 superkine on the production of the new muteins.

### Example 4. Stable production of fusion proteins based on IL-2 mutein from lentiviral transduction of HEK-293 cells adapted to suspension culture

Cloning of the expression *cassettes* of the gene constructs used for transient transfection into the lentiviral vector pL6WBlast allowed the generation of stable clones producing a subset of the fusion proteins described in the previous example:
₈₀VPPEDLIA₈₇+I92L (SEQ ID NO. 119)
₈₀VPPEDLIE₈₇+I92L (SEQ ID NO. 120)
₈₀FDPADWE₈₇+I92L (SEQ ID NO. 121)
₈₀FDPEDWE₈₇+I92L (SEQ ID NO. 122)

Clones were obtained by lentiviral transduction of HEK-293 cells adapted to grow in suspension with the gene constructs of interest derived from the pL6WBlast vector. The top five clones producing each variant were included in the study. The concentration of fusion protein in the supernatants was determined by ELISA on microtitre plates coated with an anti-IL-2 antibody. Fusion proteins were detected with a anti-human IgG antibody conjugated to a horseradish peroxidase. An IL-2/Fc (K35E) fusion protein preparation was used as a standard. For statistical analysis a one-way ANOVA was used, followed by a Tukey's multiple comparisons test (p<0.05). Figure 10 illustrates the productivity of the clones obtained. Despite the heterogeneity among the clones, presumably due to differences in the location and multiplicity of the integration of the foreign DNA into the host cell genome, a clear superiority of the clones producing the fusion proteins containing the new muteins over those producing the one including the H9 superkine was observed. One of the variants (80FDPEDWE87+I92L) (SEQ ID NO. 122) is even better produced than the others. These data reaffirmed the idea of a favourable developability profile associated with the new variants.

### Example 5. Demonstration of the increased beta-chain binding capacity and low aggregation tendency of fusion proteins containing the new muteins.

Fusion proteins (at 60 ng/mL), purified from the supernatant of HEK-293-T cells adapted for suspension growth and transfected with the corresponding gene constructs, were incubated on ELISA plates coated with the recombinant extracellular domain of the beta subunit of the receptor and detected with an anti-human Fc antibody conjugated to a horseradish peroxidase. The fusion proteins derived from the new muteins are designated according to the sequences they contain at their interface with the beta chain (segments 81-87 and position 92). Fusion proteins derived from the original IL-2 and superkine H9 were used as controls.

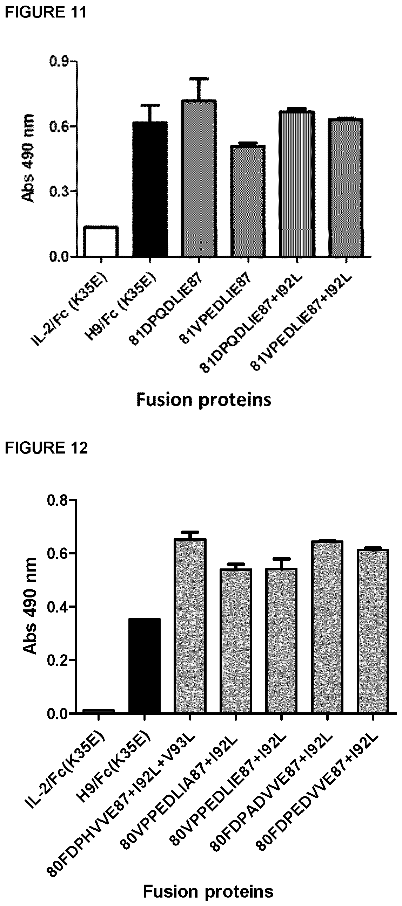

The first generation of fusion proteins (derived from the screening of the S1 library) showed an increased binding capacity to the extracellular domain of the beta subunit of the receptor relative to its original IL-2-containing counterpart (Figure 11), in the same range as that of the fusion protein based on the H9 superkine. The introduction of the I92L change in these molecules had no additional impact on the receptor beta subunit binding capacity.

Molecular exclusion chromatography profiling of the 81DPQDLIE87 (SEQ ID NO. 114) and 81VPEDLIE87 (SEQ ID NO. 115) variants revealed the presence of a major peak corresponding to the expected homodimer according to the specific dimerisation capacity of the Fc fragment (92-95%), accompanied by at least one minor peak of high molecular size aggregates (Table 2). For the versions of these fusion proteins that include the I92L change (81DPQDLIE87 +I92L (SEQ ID NO. 116) and 81VPEDLIE87+I92L (SEQ ID NO. 117)) no aggregates were evident and virtually all of the protein (>98%) was found in its homodimeric form. Despite these differences and the positive influence of the I92L mutation, all fusion proteins based on the new muteins showed a low tendency to aggregate compared to their counterpart based on the H9 superkine, which was mostly found in the form of large aggregates (Table 2).

**Table 2. Aggregation state of human Fc fusion proteins derived from first-generation muteins**

| **Fusion protein** | **Proportion of nonaggregated homodimers (%)** |
|---|---|
| **IL-2/Fc (K35E)** | 90.2 |
| **H9/Fc (K35E)** | 38 |
| **₈₁DPQDLIE₈₇** (SEQ ID NO. 114) | 94.1 |
| **₈₁VPEDLIE₈₇** (SEQ ID NO. 115) | 92.6 |
| **₈₁DPQDLIE +I92L₈₇** (SEQ ID NO. 116) | 99.2 |
| **₈₁VPEDLIE +I92L₈₇** (SEQ ID NO. 117) | 98.6 |

The second generation of fusion proteins (derived from the screening of the S2 library) showed heterogeneous behaviour in terms of aggregation levels. While the two molecules including by design the I92F change, showed a complex pattern of undesired aggregation and a minor proportion of non-aggregated homodimer (Table 3), the fusion proteins containing the muteins selected directly from the library and those designed to include a Leu residue at position 92 had a very low tendency to aggregate (> 95% homodimeric purified product) (Table 3).

**Table 3. Aggregation state of human Fc fusion proteins derived from second-generation muteins**

| **Fusion protein** | **Proportion of nonaggregated homodimers (%)** |
|---|---|
| **₈₀FDPADVVE +I92F₈₇** (SEQ ID NO. 125) | 31.6 |
| **₈₀FDPEDVVE +I92F₈₇** (SEQ ID NO. 126) | 2.5 |
| **₈₀FDPHWE +I92L₈₇** (SEQ ID NO. 118) | 97.8 |
| **₈₀VPPEDLIA +I92L₈₇** (SEQ ID NO. 119) | 97.2 |
| **₈₀VPPEDLIE +I92L₈₇** (SEQ ID NO. 120) | 97.7 |
| **₈₀FDPADVVE +I92L₈₇** (SEQ ID NO. 121) | 97.5 |
| **₈₀FDPEDVVE +I92L₈₇** (SEQ ID NO. 122) | 95.6 |

The two fusion proteins with a high tendency to aggregate, which had already been distinguished from the rest by their lower levels of secretion (Example 3), were excluded from further analysis. The remaining fusion proteins, purified from the supernatant of suspension-grown HEK-293-T cells transfected with the corresponding gene constructs, at a concentration of 30ng/mL, were incubated on ELISA plates coated with the recombinant extracellular domain of the receptor beta subunit and detected with an anti-human Fc antibody conjugated to a horseradish peroxidase. Fusion proteins derived from the original IL-2 and H9 superkine were used as controls.

The second-generation muteins tested, in addition to their low tendency to aggregate, showed an increased beta-chain binding capacity, at least 50% higher in ELISA than that of the protein based on the H9 superkine (Figure 12).

This result was corroborated by measuring the affinity and kinetic parameters of the interaction with the beta subunit by BIAcore^{™} (Table 4). The fusion proteins derived from the muteins described in the present invention had a higher affinity than those comprising unmutated IL-2 (reduction of the dissociation equilibrium constant K_{D} by more than 90 times relative to it) and superkine H9 (more than 3 times reduction in K_{D}). This increase was due to faster association kinetics (kₒₙ), coupled with a decrease in the dissociation rate constant (k_{off}).

**Table 4. BIAcore^{™} measurement of equilibrium constants and kinetic parameters of the interaction with the receptor beta chain of fusion proteins containing IL-2 derived muteins.**

| **Fusion protein** | **kₒₙ (L/mol s)** | **k_{off} (1/s)** | **KD (nmol/L)** |
|---|---|---|---|
| **IL-2/Fc(K35E)** | 3.082±0.218 x 10⁴ | 3.256±0.363 x 10⁻³ | 105.90±12 |
| **H9/Fc(K35E)** | 9.724±0.734 x 10⁴ | 3.946±1.149 x 10⁻⁴ | 4.09±1 |
| **₈₀FDPHVVE +I92L₈₇** (SEQ ID NO. 118) | 2.385±0.206 x 10⁵ | 2.660±0.184 x 10⁻⁴ | 1.12±0.02 |
| **₈₀VPPEDLIA +I92L₈₇** (SEQ ID NO. 119) | 4.357±0.339 x 10⁵ | 3.493±0.713 x 10⁻⁴ | 0.80±0.2 |
| **₈₀VPPEDLIE +I92L₈₇** (SEQ ID NO. 120) | 8.161±0.195 x 10⁵ | 3.039±0.806 x 10⁻⁴ | 0.37±0.1 |
| **₈₀FDPADVVE +I92L₈₇** (SEQ ID NO. 121) | 8.584±0.115 x 10⁵ | 4.605±0.57 x 10⁻⁴ | 0.54±0.1 |
| **₈₀FDPEDVVE +I92L₈₇** (SEQ ID NO. 122) | 9.458±0.351 x 10⁵ | 2.732±0.683 x 10⁻⁴ | 0.29±0.1 |

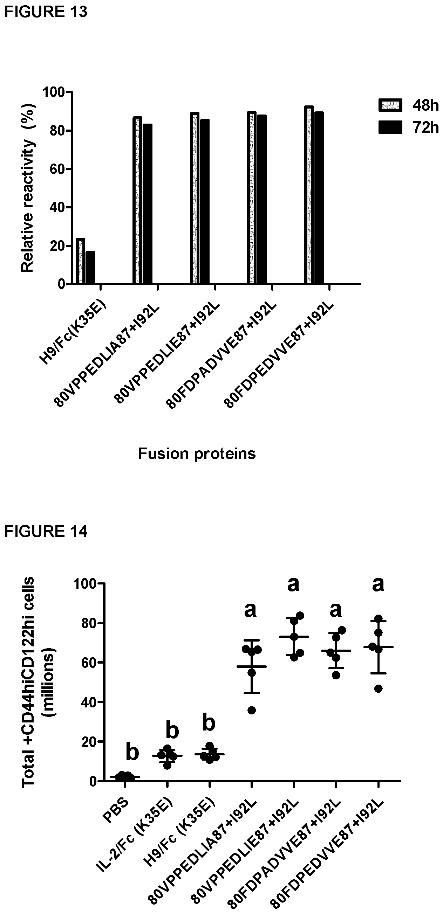

Purified proteins were incubated at 50°C for 48 and 72h, before being evaluated by ELISA on microtitre plates coated with the recombinant extracellular domain of the beta subunit of the receptor. The bound proteins were detected with a horseradish peroxidase-conjugated anti-human Fc antibody. The relative reactivity of each treated protein was calculated by taking the reactivity of the same untreated protein as a reference (100%). The H9 superkine-based fusion protein was included as a control. Figure 13 shows that the muteins of the present invention have higher thermal stability, as evidenced by the preservation of the β chain binding capacity of the fusion proteins by more than 82%. This result contrasts with the drastic loss of reactivity (greater than 76%) of the fusion protein containing the H9 superkine when subjected to similar conditions.

Altogether, the above results demonstrate that incorporation of selected mutations from our phage libraries confers IL-2 and recombinant proteins derived from it an increased beta-subunit binding capacity, coupled with a favourable developability profile in terms of expression levels, low propensity to aggregate and high thermal stability. This last group of properties represents a substantial advantage over similar molecules described in the prior art (Levin, A.M. et al., Nature. 484: 529-533, 2012).

### Example 6. Demonstration of the in vitro and in vivo superagonist capacity of novel muteins on cells displaying the dimeric IL-2 receptor beta/gamma.

To study the *in vitro* effect of fusion proteins based on IL-2 muteins, CD8+ T cells were purified from non-immunised mice and labelled with CTV. They were stimulated with each of the fusion proteins under study and the decrease in fluorescence in the cells was assessed by flow cytometry as an indicator of the ability of the proteins to induce proliferation of this cell population. Unstimulated cells and cells treated with the fusion proteins containing the original IL-2 and superkine H9 were used as controls. The fusion proteins containing the four new IL-2 muteins studied induced proliferation of 93-96% of CD8+ T cells, with similar behaviour to the H9 superkine-based protein (table 5). In contrast, the human IL-2/Fc fusion protein had a moderate effect, inducing proliferation of only 55.3% of the cells. These results demonstrated the *in vitro* superagonist capacity of the new variants on cells carrying the dimeric beta/gamma IL-2 receptor.

**Table 5. In vitro expansion of CD8+ T cells induced by fusion proteins containing the IL-2 muteins**

| **Fusion protein** | **Proportion of proliferating purified CD8+ T cells (%)** |
|---|---|
| **IL-2/Fc (K35E)** | 55.3 |
| **H9/Fc (K35E)** | 94.0 |
| **₈₀VPPEDLIA +I92L₈₇** (SEQ ID NO. 119) | 94.3 |
| **₈₀VPPEDLIE +I92L₈₇** (SEQ ID NO. 120) | 93.5 |
| **₈₀FDPADVVE +I92L₈₇** (SEQ ID NO. 121) | 94.5 |
| **₈₀FDPEDVVE +I92L₈₇** (SEQ ID NO. 122) | 95.9 |

For *in vivo* experiments, LALA Fc-containing versions of the fusion proteins (with the L234A and L235A mutations) (SEQ ID NO. 123) were used to prevent their binding to Fc gamma receptors and the occurrence of Fc gamma re3ceptor-mediated immunological effects beyond the action of the muteins. These new fusion proteins were designated as:
₈₀VPPEDLIA₈₇+I92L/LALA (SEQ ID NO. 119)
₈₀VPPEDLIE₈₇+I92L/LALA (SEQ ID NO. 120)
₈₀FDPADWE₈₇+I92L/LALA (SEQ ID NO. 121)
₈₀FDPEDWE₈₇+I92L/LALA (SEQ ID NO. 122)

In contrast to the previous scenario, *in vivo* stimulation experiments showed a greater effect of the fusion proteins based on the novel muteins compared to their counterpart containing superkine H9. After daily intraperitoneal treatment for four consecutive days of each group of animals with 5.6 µg of each of the fusion proteins, on the fifth day the mice were sacrificed, their spleens were removed, macerated and the cells were analysed by flow cytometry. For statistical analysis, a one-way ANOVA was used, followed by a Tukey's multiple comparisons test (p< 0.05).

The number of activated CD8+ T cells (CD8+CD44hiCD122hi) was significantly increased in the groups treated with the fusion proteins containing the new muteins, compared to animals treated with IL-2/FcLALA, H9/FcLALA and the control group that received only phosphate buffered saline (PBS) (Figure 14).

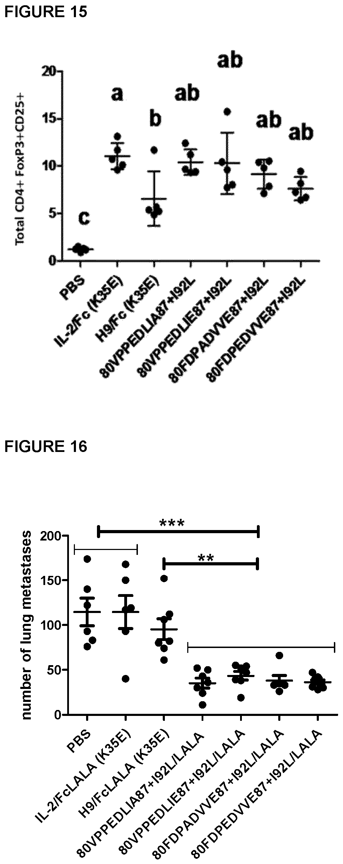

In contrast, the regulatory T cell population (CD4+CD25+FoxP3+), although expanded in animals treated with all fusion proteins compared to the control group, showed no difference between animals that received the new fusion proteins (Figure 15) and those inoculated with the IL-2/Fc and H9/Fc proteins used as controls. These results demonstrate the selective advantage of the new muteins with increased affinity for the beta subunit of the receptor for *in vivo* activation of immune system effector cells carrying the dimeric beta/gamma receptor, over IL-2 and the H9 superkine described in the prior art. Their increased effector potential is not accompanied by an increase in regulatory function, so the net balance should enhance the effector functions of the immune system.

These results demonstrate the selective advantage of novel muteins with increased affinity for the beta subunit of the receptor for *in vivo* activation of immune effector cells carrying the dimeric beta/gamma receptor over IL-2 and H9 superkine described in the prior art.

### Example 7. Demonstration of the anti-tumour effect of new muteins

The anti-tumour effect of fusion proteins based on the novel human FcLALA-fused muteins was studied in the experimental melanoma metastasis model MB16/F0. An amount of 100,000 cells of this tumour line were inoculated via the tail vein of C57BL/6 mice and each group of animals was then treated with an intraperitoneal injection of 5.6 µg of each of the fusion proteins containing the IL-2 muteins. Treatment with the fusion proteins was repeated every 24 h until day 5. Lung metastases were counted 21 days after the initial inoculation. For statistical analysis, a one-tailed ANOVA was used, followed by a Tukey's multiple comparisons test (p< 0.05).

Lung metastases counts were significantly reduced in animals treated with the fusion proteins containing the new muteins compared to animals in the control group treated with PBS and those receiving IL-2/FcLALA or H9/FcLALA proteins (Figure 16). The anti-tumour effect of the new muteins was thus demonstrated, in a scenario where neither the original IL-2 nor the H9 superkine in an equivalent fusion protein format has an anti-metastatic effect. These results corroborated the enhanced *in vivo* biological activity of the new superagonist muteins in comparison not only with the original IL-2, but also to a superagonist described in the prior art.

### Example 8. Combination of mutations identified at the interface with the beta chain of the receptor with substitutions known to break the interaction with the alpha chain.

A human Fc fusion protein (LALA version) was designed and constructed containing a variant of IL-2 with the sequence ₈₀VPPEDLIE₈₇+I92L at the interface with the beta chain of the receptor and the mutations R38A, F42A, Y45A and E62A, known to break the interaction with the alpha chain in a variant called IL-2 no-alpha and additionally the K35E mutation whose effects on increased IL-2 secretion are known (Rojas, G et al., Sci. Reports. 9: 800, 2019). The above set of mutations is described in SEQ ID NO. 4 of patent EP 3 543 252 B1. This protein was produced in the transient transfection system of HEK293-T cells adapted to grow in suspension described above.

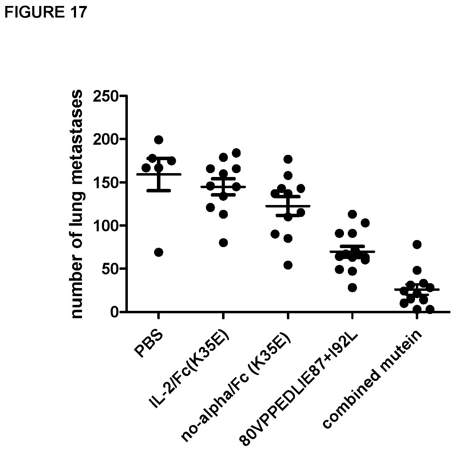

The anti-tumour effect of fusion protein based on the combined mutein (SEQ ID NO. 124) was studied in the experimental melanoma metastasis model MB16/F0, compared to fusion proteins containing only the changes at one of the interfaces. An amount of 100,000 tumour cells were inoculated via the tail vein of C57BL/6 mice and each group of animals was then treated with an intraperitoneal injection of 1.9 µg of each of the fusion proteins containing the IL-2 muteins. Treatment with the fusion proteins was repeated every 24h until day 5. Lung metastases were counted 21 days after the initial inoculation. For statistical analysis, a one-tailed ANOVA was used, followed by a Tukey's multiple comparisons test (p< 0.05). Figure 17 shows the reduced metastases formation in animals treated with the combined mutein-based fusion protein compared to those that received PBS, the control fusion protein containing the original IL-2 and fusion proteins containing changes in only one of the two interfaces (no-alpha mutein and ₈₀VPPEDLIE₈₇+I92L variant). These results demonstrate the utility of combining the mutations at the interface with the IL-2 receptor beta chain, described as part of the present invention, with other substitutions known to affect IL-2 properties.

## Claims

1. A mutein derived from human interleukin-2 (IL-2) comprising: amino acid substitutions in at least two of positions 81, 83, 84 and 87 of the primary amino acid sequence of IL-2 resulting in a net negative charge of segment 81-87 equal to or greater than -2 and optionally containing the I92L change.

2. The mutein according to claim 1 wherein positions 81 and 83 are replaced by amino acids which are selected from the group comprising: Lys, His, Asp, Glu, Ala, Ala, Asn, Gly, Gln, Ile, Leu, Met, Phe, Pro, Ser, Thr, Val, Trp and Tyr.

3. The mutein according to any one of claims 1 and 2 wherein positions 84 and 87 are replaced by amino acids selected from the group comprising: Asp, Glu, Ala, Asn, Gly, Gln, Ile, Leu, Met, Phe, Pro, Ser, Thr, Val, Trp and Tyr.

4. The mutein according to any one of claims 1-3 wherein position 80 is replaced by amino acids selected from the group comprising: Phe, Val, Met and Ile.

5. The mutein according to any one of claims 1-4 wherein position 82 is replaced by Ala.

6. Mutein according to any one of claims 1-5 wherein position 85 is replaced by amino acids selected from the group comprising: Ile, Val and Met.

7. The mutein according to any one of claims 1-6 wherein position 86 is replaced by Val.

8. The mutein according to any one of claims 1-7 wherein position 89 is replaced by amino acids selected from the group comprising: Leu, and Met.

9. Mutein according to any one of claims 1-3 wherein position 93 is replaced by amino acids selected from the group comprising: Ile and Leu.

10. The mutein according to any one of claims 1-9 wherein position 35 is replaced by amino acids selected from the group comprising: Glu, Asp and Gln.

11. The mutein according to any one of claims 1-10 wherein mutations R38A, F42A, Y45A and E62A are introduced.

12. The mutein according to any one of claims 1-11 **characterised by** being fused to any one of the proteins selected from the group comprising:
- capsid proteins of filamentous phage;
- albumin;
- the Fc region of the antibodies;
- complete antibodies;
- antibody fragments that include its variable domains and
- other cytokines

13. A pharmaceutical composition **characterised by** comprising the mutein of any one of claims 1-12 in a concentration range of 1 mg/ml to 20 mg/ml and a pharmaceutically suitable excipient.

14. Use of the muteins of any one of claims 1-13 for therapeutic purposes in the treatment of cancer and immunodeficiencies.

15. Use of the muteins of any one of claims 1-13 for *in vitro* expansion of T and NK cells for adoptive transfer therapies.
